# EUROPÄISCHE PATENTSCHRIFT

(11) **EP 0 667 117 B1**
(45) Veröffentlichungstag und Bekanntmachung des Hinweises auf die Patenterteilung: **22.04.1998**
(21) Anmeldenummer: 94100413.7
(22) Anmeldetag: 13.01.1994
(51) Int. Cl.: A61B 5/103

(54) **Apparat und rechnergestütztes medizinisches Untersuchungsverfahren zur Verarbeitung physikalischer Bildparameter der Hautoberfläche**
Computer aided medical diagnosis system for processing physical image parameter of the skin surface
Système diagnostic à usage médical pour le traitement de paramètre d'image physique de la surface de la peau

(43) Veröffentlichungstag der Anmeldung: 16.08.1995
(73) Patentinhaber: Voigt, Holger, Dr. med., D-24568 Kaltenkirchen/Holstein (DE)
(72) Erfinder: Voigt, Holger, Dr. med., D-24568 Kaltenkirchen/Holstein (DE)

(56) Entgegenhaltungen:
- GB-A- 2 159 943
- US-A- 4 807 163
- US-A- 4 930 872

## Beschreibung

Die Erfindung betrifft einen Untersuchungsstand zur Durchführung eines rechnergestützten medizinischen Untersuchungsverfahrens zur Erfassung, Speicherung und rechnerischen Verarbeitung physikalischer Bildparameter der Hautoberfläche, bei dem eine rechnerische Bildanalyse gleichzeitig an mehreren bis zahlreichen abgrenzbaren Hautstrukturmerkmalen unter quantitativ reproduzierbarer Positionierung einer zu untersuchenden Person zum Zwecke der Erfassung von Änderungen im Zeitverlauf seriell aufeinanderfolgend durchgeführt wird.

Die visuelle Erfassung von abgrenzbaren Strukturmerkmalen der Hautoberfläche erfolgt in der medizinischen Diagnostik durch direkte Inaugenscheinnahme anläßlich der ärztlichen Untersuchung. Der Ablauf dieser Vorgehensweise orientiert sich dabei jeweils an einem prägnanten, abgrenzbaren Einzelbefund, dessen Strukturanalyse zum Zwecke der diagnostischen Einordnung durch empirischen Strukturvergleich auf der Basis medizinischen Sachkenntnisstandes durchgeführt wird. Ergänzend werden für diesen subjektiven Erfassungsvorgang objektive Meßparameter, wie z.B. bei einer metrischen Größenbestimmung durch Ausmessung des Durchmessers einer abgrenzbaren Hautoberflächenstruktur unter Verwendung von Maßstabsskalen, herangezogen.

Der in den letzten Jahren erfolgte Einsatz von rechnergestützten Systemen zur Bilderfassung und Bildverarbeitung am Hautorgan verfolgt die Zielsetzung einer Optimierung und Vereinfachung der Diagnosestellung einer jeweils einzelnen zu analysierenden Hautveränderung. Anhand der zu untersuchenden Einzelstruktur werden dabei objektive Meßparameter erfaßt, die eine zutreffende diagnostische Einordnung der in Frage stehenden Strukturveränderung der Hautoberfläche ermöglichen. Über das bei diesen Verfahren benutzte Rechnerprogramm können zusätzlich weitere Parameter mitverarbeitet werden, die nicht direkt durch den visuellen Untersuchungsgang der abklärungsbedürftigen Hautveränderung erfaßbar sind, sondern andere biologische Merkmale, z.B. Augenfarbe oder Daten aus der Krankengeschichte, berücksichtigen (Green A, Martin N, McKenzie G, Pfitzner J, Quintarelli F, Thomas BW, O'Rourke M, Knight N: Computer image analysis of pigmented skin lesions, Melanoma Research, 1 (1991) 231 - 236; Cascinelli N, Ferrario M, Bufalino R, Zurrida S, Galimberti V, Mascheroni L, Bartoli C, Clemente C: Results obtained by using a computerized image analysis system designed as an aid to diagnosis of cutaneous melanoma, Melanoma Research, 2 (1992) 163 - 170).

Obwohl mit den beschriebenen Ansätzen eine objektive Erfassung von Merkmalen einzelner Hautoberflächenstrukturen durchgeführt werden kann, ist es bislang nicht möglich, in einem einzigen Untersuchungsgang Parameter mehrerer bis zahlreicher Hautoberflächenstrukturen gleichzeitig zu erfassen und rechnerisch zum Zwecke eines zeitbezogenen Strukturvergleichs zu verarbeiten.

Das im Patent GB-A-2 159 943 dargestellte Verfahren ermöglicht die Erfassung und rechnerische Auswertung von zeitbezogenen Ausdehnungs- und Farbveränderungen einer Körperabschnittskontur nach Aufnahme eines über eine Videokamera erfaßten Oberflächenbildes. Dabei erfolgt die rechnerische Bildvergleichsanalyse hinsichtlich der Veränderung von Körperdimensionen, z.B. vor und nach einer Behandlung oder Diät, durch Bildüberlagerung sowie arithmetischer Umfangsbestimmungen nach Messung der Durchmesser von Körperachsenquerschnitten unter Annahme geometrischer Querschnittsbeziehungen, wobei Meßparameter aus Aufnahmesets unterschiedlicher Aufnahmeprojektion verarbeitet werden, die aus jeweils 4 Bildern in Vorderansicht, Rückansicht und zwei Seitenansichten bestehen. Mit diesem Verfahren ist es allerdings nicht möglich, strukturierte, einzeln abgrenzbare Oberflächenmerkmale, z.B. Hautveränderungen, in ihrer Gesamtheit zu identifizieren, zu zählen, gleichzeitig hinsichtlich ihrer Position und ihrer bildbezogenen physikalischen Merkmale zu erfassen und vergleichend quantitativ zu analysieren. Eine quantitative Positionierungsreproduzierbarkeit zum Ausschluß von Einflüssen aufgrund unterschiedlicher Aufnahmeposition, Raumstellung und Körperhaltung als Voraussetzung für eine quantitativ-vergleichende Bildanalyse ist nach dem Verfahren GB-A-2 159 943 nicht sichergestellt, weshalb davon auszugehen ist, daß durch Positionsverzerrungen eine quantitative Bildanalyse mit diesem Verfahren lediglich näherungsweise und eine für diagnostische Belange wichtige Positions- und Mustererkennung mit Ermittlung quantitativer Größenparameter einzelner in demselben Bild vorhandener Merkmale überhaupt nicht möglich ist.

Die Erfindung bezweckt die Herstellung einer zur Durchführung des Untersuchungsverfahrens erforderlichen quantitativ reproduzierbaren Positionierung einer zu untersuchenden Person.

Diese Aufgabe wird erfindungsmäßig durch die Ansprüche 1 - 8 gelöst, wobei die für Verlaufsuntersuchungen erforderliche quantitativ reproduzierbare Positionierung einer zu untersuchenden Person durch den in den Ansprüchen 1 - 4 dargelegten Untersuchungsstand und die Verwendung des Untersuchungsstandes im Untersuchungsverfahren in den Ansprüchen 5 - 8 dargelegt sind. Dabei wird die verfahrenswesentliche reproduzierbare Positionierung einer Untersuchungsperson zusätzlich durch Nutzung einer bildverarbeitenden Software sichergestellt.

Der Verwendung des Untersuchungsstandes ermöglicht im Unterschied zu den von Green sowie Cascinelli angegebenen Verfahren die Durchführung des Untersuchungsverfahrens unter Ausdehnung des jeweiligen Bilderfassungsbereiches von dem einer Einzelstruktur auf einen individuell wählbaren Ausschnitt der Hautoberfläche mit der Möglichkeit der gleichzeitigen Erfassung und rechnerischen Verarbeitung von physikalischen Bildparametern mehrerer bis zahlreicher abgrenzbarer Einzelstrukturen und deren vergleichender Analyse zum Zwecke einer Verlaufsbeurteilung durch Erkennung von Veränderungen zuvor erfaßter Parameter, wobei neu hinzugetretene und im Verlauf als fehlend erkannte Strukturmerkmale identifiziert, gezählt und hinsichtlich ihrer Position im Bild markiert werden.

Die Erfindung kann in der Diagnostik, Verlaufsbeobachtung und Nachsorge von Krebserkrankungen und anderen Erkrankungen, die in der Haut entstehen oder an der Hautoberfläche erkennbar sind, eingesetzt werden. Sie ist inbesondere im Bereich der Krebsfrüherkennung zur Erfassung und Verlaufsbeobachtung von Risikogruppen mit zahlreichen Hautpigmentmälern sowie bei Personengruppen mit zahlreichen Hautstrukturveränderungen unterschiedlicher Ursächlichkeit im Sinne einer Reihenuntersuchung geeignet.

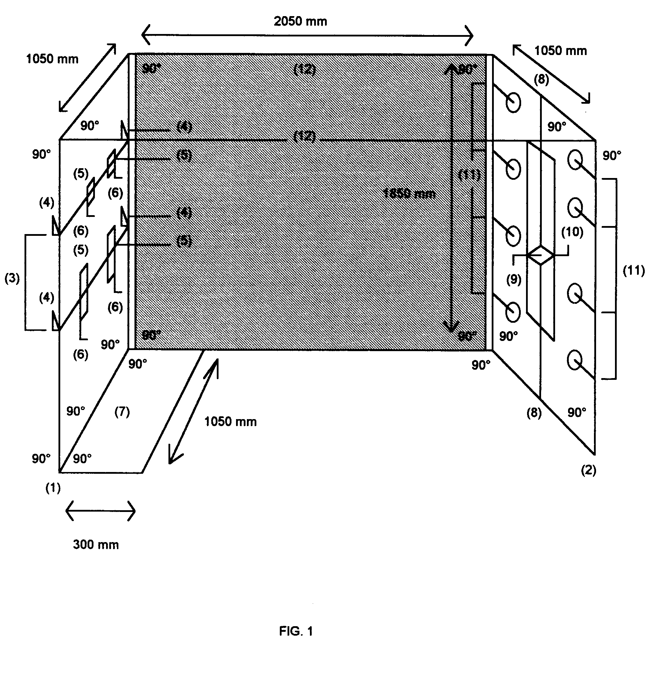

Der Untersuchungsstand ist schematisch in Abbildung 1 dargestellt.

Die Ebene der Positionserfassung einer zu untersuchenden Person wird als Positionsebene (1), die der Kamera- und Beleuchtungshalterung als Kamera-/Beleuchtungsebene (2) bezeichnet. Beide Ebenen befinden sich über zwei rechtwinklig angeordnete Verbindungsleisten (12) eines Rahmenstativs miteinander verbunden hintereinander in gerader Linie angeordnet in einem konstanten Abstand von 2050 mm.

Die Positionsebene (1) besteht aus einem Rahmenstativ, in welches schwarz gefärbte Kunststoffplatten eingelassen sind. Die Höhe des Rahmenstativs beträgt 1850 mm, die Breite 1050 mm. Der Rahmen weist in vertikaler Anordnung im Abstand von jeweils 50 mm beidseits angeordnete Bohrlöcher auf. In diese Bohrlöcher werden horizontale Schiebereglerhalterungen (3) eingefügt und beidseits mit jeweils einem Schließhaken (4) auf gleicher Höhe des Rahmens fixiert. Die Schiebereglerhalterungen (3) bestehen aus einer Führungsleiste mit Maßstabsskala, die jeweils 2 Schieberegler in einer Rollenzugführung über die Leiste führt. Beide Schieberegler weisen seitliche, vertikal stehende Begrenzungsplatten (5) auf. Über den horizontalen Rollenzug können die Schieberegler horizontal auf der Leiste verschoben werden, wobei sich eine jeweils nach den Außenseiten identische und nach der Mitte zentrierte Position ergibt. Die Begrenzungsplatten (5) der Schieberegler können jeweils durch einen Klemmhebel (6) auf der Führungsleiste fixiert werden, sobald die gewünschte Position erreicht ist. Die nach beiden Seiten identische Position ist an der Maßstabsskala ablesbar.

Bei der Bilderfassung wird die äußere seitliche Körperbegrenzung einer zentriert im Untersuchungsstand auf einer zwischen Positionsebene (1) und Kamera-/Beleuchtungsebene (2) direkt vor der Positionsebene (1) befindlichen Standfläche (7) der Größe 1050 mm x 300 mm aufrecht stehenden Person durch die jeweils in Hüft- und Brusthöhe angeordneten Schieberegler eingestellt und unverändert bei Vergleichsuntersuchungen im Zeitverlauf übernommen.

Die Kamera-/Beleuchtungsebene (2) weist eine vertikal angeordnete, zentrierte Führungsleiste (8) mit Maßstabsskala auf, über die in vertikaler Führung eine Kamerahalterung (9) mit horizontal angeordneter Grundplatte zur Aufnahme und Fixierung einer Kamera eingestellt werden kann. Die Position der Kamerahalterung (9) ist in vertikaler Richtung verschiebbar, über einen Klemmhebel (10) fixierbar und an der Maßstabsskala der Führungsleiste (8) ablesbar.

Bei der Bilderfassung wird die Einstellung der Kamerahalterung (9) für Vergleichsuntersuchungen im Zeitverlauf unverändert übernommen.

An den seitlichen Rahmenleisten der Kamera-/Beleuchtungsebene (2) befinden sich in Abständen von jeweils 200 mm Bohrlöcher zur Aufnahme und Fixierung von Halterungen von Leuchtstrahlern (11). Die Position der Leuchtstrahler (11) bleibt für Vergleichsuntersuchungen unverändert. Insgesamt können auf jeder Seite bis zu 4 Leuchtstrahler (11) an der seitlichen Rahmenführung befestigt werden.

Zum Zwecke der Stabilisierung des Standes und Abstandsfixierung von Positionsebene (1) und Kamera-/Beleuchtungsebene (2) werden diese unter Verwendung von horizontal angeordneten Verbindungsleisten (12) einer Länge von 2050 mm derart zusammengefügt, daß die oberen Begrenzungen von Positionsebene (1) und Kamera-/Beleuchtungsebene (2) mit mindestens jeweils 2 randständigen Verbindungsleisten (12) rechtwinklig verbunden sind.

Für den Vorgang der Bilderfassung und Bildverarbeitung werden eine hochauflösende CCD-Videokamera, ein Bildverarbeitungsrechner mit Drucker sowie eine speziell für dieses Untersuchungsverfahren entwickelte Bildverarbeitungssoftware verwendet. Die bildverarbeitende Software überprüft dabei durch Abstandsmessung zwischen Bildpunktebenen im Bildausschnitt erfaßter unveränderlicher Konturen, z.B. der Begrenzungsplatten (5) der Schiebereglerhalterungen (3), die Reproduzierbarkeit der Positionierung quantitativ und führt eine rechnerische Speicherung und Verarbeitung der erfaßten physikalischen Bildparameter in der Weise durch, daß neben der Erfassung und Speicherung des Hautoberflächenbildes Anzahl, Anordnung, Flächengröße und physikalische Bildstruktur sämtlicher im Bildausschnittsbereich enthaltener, abgrenzbarer Einzelstrukturen erfaßt, im Bild markiert, gespeichert und im Zeitverlauf vergleichend analysiert werden, wobei durch Bildvergleich nachweisbare Veränderungen der physikalischen Bildparameter identifiziert, rechnerisch erfaßt, im Bild optisch markiert und rechnerisch auf Speichermedien hinterlegt werden.

Der verfahrenstechnische Ablauf des mit Hilfe des Untersuchungsstandes durchgeführten Untersuchungsverfahrens besteht aus einer Vorbereitung des Untersuchungsstandes und einer Untersuchung.

Die Vorbereitung des Untersuchungsstandes besteht darin, daß im Bereich der Positionsebene (1) die Schiebereglerhalterungen (3) in ihrer vertikalen Anordnung auf die Brust- und Hüfthöhe der zu untersuchenden Person eingestellt und über Schließhaken (4) am Rahmenstativ fixiert werden. Die vertikale Positionierung der Schiebereglerhalterungen (3) ist durch die Anordnung der Bohrlöcher des Rahmens ablesbar und wird für Folgeuntersuchungen dokumentiert. Im Bereich der Kamera-/Beleuchtungsebene (2) wird die Kamerahalterung (9) auf der vertikalen Führungsleiste (8) in ihrer Höhe so justiert, daß bei geöffneter Kamerablende der gewählte Ausschnittsbereich vollständig erfaßt ist. Die vertikale Einstellung der Kamerahalterung (9) wird über einen Klemmhebel (10) auf der Führungsleiste (8) fixiert und ihre Position an der auf der Führungsleiste (8) angebrachten Maßstabsskala abgelesen und dokumentiert. Die im Bereich des Rahmenstativs der Kamera-/Beleuchtungsebene (2) angeordneten Leuchtstrahler (11) werden an den am Rahmenstativ befestigten Halterungen fixiert und horizontal ausgerichtet. Ihre Anordnung und Anzahl bleibt für Vergleichsuntersuchungen unverändert.

Der Untersuchungsablauf erfolgt in der Weise, daß eine zu untersuchende Person zum Zwecke einer im Zeitverlauf quantitativ reproduzierbaren Position von einer Assistenzperson im Untersuchungsstand positioniert wird, wobei bei aufrechter Körperhaltung die zu untersuchende Person auf einer Standfläche (7) vor der Positionsebene (1) zentriert steht und die auf den Schiebereglerhalterungen (3) befindlichen Schieberegler mit ihren vertikal stehenden seitlichen Begrenzungsplatten (5) in Brust- und Hüfthöhe durch Verschieben auf der horizontalen Schiebereglerhalterung (3) von der Assistenzperson so eingestellt werden, daß die Begrenzungsplatten (5) abschließenden, flächigen Hautkontakt haben. In dieser Position werden die Schiebereglerplatten (5) auf der Schiebereglerhalterung (3) mittels der unter den Schiebereglerplatten (5) befindlichen Klemmhebel (6) fixiert und ihre Einstellung von einer auf der Schiebereglerhalterung (3) befindlichen Maßstabsskala abgelesen und dokumentiert. In dieser Position erfolgt anschließend die Bilderfassung über die in der Kamera-/Beleuchtungsebene (2) in der Kamerahalterung (9) eingepaßte Videokamera. Dabei werden die im Bildausschnitt dargestellten und von einer Videokamera erfaßten, aus einer Vielzahl von Bildpunkten bestehenden Begrenzungslinien der Schiebereglerplatten (5) von einer für die rechnerische Auswertung eingesetzten Bildverarbeitungssoftware als Bezugsbildpunkte rechnerisch verarbeitet, wobei durch Bestimmung von Bildpunktabständen zwischen den beiderseits angeordneten Schiebereglerplatten (5) einerseits sowie zwischen den Schiebereglerplatten (5) und den im Bild erfaßten anatomischen Konturlinien einer zu untersuchenden Person andererseits die Reproduzierbarkeit der Position einer zu untersuchenden Person sichergestellt wird. Das unter quantitativ reproduzierbaren Untersuchungsbedingungen erfaßte Bild wird über Rechner und Bildverarbeitungssoftware auf einem Speichermedium gespeichert. Die weitere Bildverarbeitung erfolgt am Rechner.

Zum Zwecke der Vergleichsuntersuchung im Zeitverlauf wird die Vorbereitung des Untersuchungsstandes in gleicher Weise vorgenommen, wobei identische Positionen der Schiebereglerhalterung (3), Schiebereglerplatten (5), Kamerahalterung (9), Blendenstellung der Kamera, Bildausschnitts- und Begrenzungswerte des Kamerabildes sowie der Position der Leuchtstrahler (11) eingestellt werden. Die zu untersuchende Person wird anschließend von einer Assistenzperson in den voreingestellten Untersuchungsstand positioniert.

Die Bilderfassung, Bildspeicherung und rechnerische Bildverarbeitung erfolgt bei der Zeitverlaufsuntersuchung in gleicher Weise wie bei der Erstuntersuchung. Zusätzlich werden durch Vergleich von jeweils zwei auf entsprechenden Speichermedien gespeicherten Bildern Änderungen der physikalischen Bildparameter von der bildverarbeitenden Software identifiziert, rechnerisch vergleichend verarbeitet, gespeichert und auf dem Hautoberflächenbild in der Weise markiert, daß neu hinzugetretene, in ihrer Größe, Anordnung oder physikalischen Bildstruktur veränderte, größengleiche sowie die Position im Zeitverlauf als fehlend identifizierter Einzelstrukturen der Hautoberfläche optisch markiert werden.

Sowohl die erfaßten Hautoberflächenbilder als auch die erstellten Berechnungsprotokolle werden über einen angeschlossenen Drucker für Dokumentationszwecke ausgedruckt.

## Patentansprüche

1. Untersuchungsstand mit Mitteln zur Erfassung, Speicherung und rechnerischen Verarbeitung physikalischer Bildparameter der Hautoberfläche, mit
- Mitteln zum Positionieren (3, 4, 5) zur Erzielung einer für Zeitverlaufsuntersuchungen quantitativ reproduzierbaren Körperposition einer zu untersuchenden Person,
- einer Videokamera zum Erfassen der physikalischen Bildparameter eines Hautoberflächenbereiches,
- Mitteln zum Speichern und Verarbeiten der Bildparameter,
wobei zur Erzielung quantitativ reproduzierbarer Körperpositionen einer zu untersuchenden Person Verbindungsleisten zu einem Rahmenstativ derart zusammengefügt sind, daß eine Positionsebene (1) und eine Kamera-/Beleuchtungsebene (2) in definiertem Abstand hintereinander angeordnet und über rechtwinklig mit Positionsebene (1) und Kamera-/Beleuchtungsebene (2) verbundene Stativleisten (12) verbunden sind, wobei die Mittel zum Positionieren einer in einer quantitativ reproduzierbaren Position zu untersuchenden Person im Bereich der Positionsebene (1) vertikal in ihrer Anordnungshöhe verstellbare und skalierte Schiebereglerhalterungen (3) sowie horizontal verstellbare, auf die seitliche Körperbegrenzung einer zu untersuchenden Person einstellbare Begrenzungsplatten (5) enthalten, deren Randbegrenzungen quantitativ erfaßt und von einer bildverarbeitenden Software in einem von einer Videokamera aufgenommenen Bild zur Herstellung und Überprüfung von Positionsreproduzierbarkeit verarbeitet werden, und im Bereich der Kamera-/Beleuchtungsebene (2) auf einer vertikal und zentriert angeordneten skalierten Führungsleiste (8) eine vertikal in ihrer Anordnungshöhe verstellbare Kamerahalterung (9) sowie am Rahmenstativ der Kamera-/Beleuchtungsebene (2) vertikal in ihrer Anordnungshöhe verstellbare Halterungen zur Aufnahme und Fixierung von Beleuchtungsstrahlern (11) aufweisen.

2. Untersuchungsstand nach Anspruch 1, der zur Erzielung quantitativ reproduzierbarer Körperpositionen einer zu untersuchenden Person im Bereich der Positionsebene (1) vertikal in ihrer Anordnungshöhe verstellbare und mit Schließhaken (4) am Rahmenstativ fixierbare skalierte Schiebereglerhalterungen (3) aufweist, deren Schieberegler mit seitlichen Begrenzungsplatten (5) versehen sind, deren horizontale Position auf der Schiebereglerhalterung (3) verschiebbar und mit Klemmhebeln (6) an dieser fixierbar ist.

3. Untersuchungsstand nach Anspruch 1, der zur Erzielung quantitativ reproduzierbarer Untersuchungsbedingungen im Bereich der Kamera-/Beleuchtungsebene (2) eine vertikal in ihrer Anordnungshöhe auf einer skalierten Führungsleiste (8) verschiebbare Kamerahalterung (9) aufweist, deren Position mit einem Klemmhebel (10) an der Führungsleiste (8) fixierbar ist.

4. Untersuchungsstand nach Anspruch 1, der zur Stellungspositionierung einer zu untersuchenden Person eine im Standboden eingelassene Standfläche (7) aufweist, die zwischen Positionsebene (1) und Kamera-Beleuchtungsebene (2) unmittelbar vor der Positionsebene (1) angeordnet ist.

5. Verwendung eines Untersuchungsstandes nach einem der Ansprüche 1 - 4 in einem rechnergestützten medizinischen Untersuchungsverfahren zur Erfassung, Speicherung und rechnerischen Verarbeitung von über eine Videokamera erfaßten Bildern einer Körperoberfläche eines Menschen, wobei im verfahrenstechnischen Ablauf eine zu untersuchende Person unter Verwendung von quantitativ reproduzierbaren Positionierungsmitteln in einer für Zeitverlaufsuntersuchungen quantitativ reproduzierbaren Position positioniert wird, wobei für die rechnerische Bildverarbeitung ein quantitativ über die Positionierungsmittel bezogener reproduzierbarer Hautoberflächenbereich ausgewählt wird, dessen physikalische Bildparameter von einer Videokamera erfaßt, rechnerisch gespeichert und verarbeitet werden, wobei durch Nutzung einer bildverarbeitenden Software die quantitative Positionierungsreproduzierbarkeit überprüft und eine rechnerische Bildanalyse gleichzeitig an mehreren bis zahlreichen abgrenzbaren Hautstrukturmerkmalen des erfaßten Hautoberflächenbildes in der Weise vorgenommen wird, daß die abgrenzbaren Hautstrukturen hinsichtlich ihrer Anzahl, Anordnung, Flächengröße und physikalischen Bildstruktur sowie ihrer im Zeitverlauf erfolgenden Veränderung von Anzahl, Anordnung, Flächengröße und physikalischer Bildstruktur erfaßt, im Bild optisch markiert, gespeichert, rechnerisch verarbeitet und im Vergleich mit zeitlich zuvor erfaßten und gespeicherten Bildern optisch und rechnerisch analysiert werden, wobei im Zeitverlauf neu hinzugetretene Einzelstrukturen identifiziert und diese sowie im Verlauf größenveränderte, größenunveränderte, anordnungsveränderte, in ihrer physikalischen Bildstruktur veränderte sowie die Positionen im Verlauf als fehlend identifizierter Einzelstrukturen dadurch markiert werden, daß sie im Bild optisch hervorgehoben werden.

6. Verwendung nach Anspruch 5, wobei eine rechnerische Bildanalyse durch vergleichende optische und rechnerische Auswertung im Zeitverlauf seriell aufeinanderfolgend durchgeführt wird.

7. Verwendung nach Anspruch 5, wobei eine optische Markierung rechnerisch verarbeiteter abgrenzbarer Einzelstrukturen der Hautoberfläche durch Numerierung, farbliche Hervorhebung oder graphische Einfassung oder eine Kombination dieser Markierungsverfahren durchgeführt wird.

8. Verwendung nach Anspruch 5, wobei optische und rechnerische Analysen physikalischer Bildparameter erfaßter Hautoberflächenbilder auf Speichermedien hinterlegt und über einen Drucker graphisch und rechnerisch als Meßprotokoll ausgegeben werden.

## Claims

1. Examination stand with means for the recording, storage, and arithmetical processing of physical image parameters of the skin surface, with
- positioning means (3, 4, 5) for the attainment of a body position of a person to be examined, which is quantitatively reproducible with respect to the course of time,
- a video camera for the capturing of the physical image parameters of a skin surface area,
- means for the storing and processing of the image parameters,
wherein for the attainment of quantitatively reproducible body positions of a person to be examined braces are joined together as a framework in a way that a position plane (1) and a camera-/illumination plane (2) are placed in a row at a defined distance and are connected through framework braces (12), which are joined rectangularly with the position plane (1) and the camera-/illumination plane (2), wherein the means for the positioning of a person to be examined in a quantitatively reproducible position are containing in the area of the position plane (1) slider mountings (3), which are vertically adjustable in their height position and scaled, as well as border plates (5), which are horizontally adjustable to the lateral extent of the body contour of a person to be examined, and wherein the border contours of said plates are being quantitatively assessed and are being processed within a video-camera taken image through an image processing software in order to attain and to check positioning reproducibility, and are further containing in the area of the camera-/illumination plane (2) a camera mounting (9), which is vertically adjustable in its height position on a vertical and centred scaled guiding brace (8), as well as adjustable mountings for the placement and fixation of illumination lamps (11) to the framework of the camera-/illumination plane (2), which are vertically adjustable in their height position.

2. Examination stand according to claim 1, comprising in the area of the position plane (1) scaled slider mountings (3) for the attainment of quantitatively reproducible body positions of a person to be examined, which are vertically adjustable in their height position and are fixable to the framework through latches (4), wherein the sliders of said slider mountings (3) are containing lateral border plates (5), the horizontal positions of which are being movable on the slider mounting (3), and which are being fixable to said slider mounting (3) through clasps (6).

3. Examination stand according to claim 1, comprising in the area of the camera-/illumination plane (2) a camera mounting (9) for the attainment of quantitatively reproducible examination conditions, which is being vertically movable in its height position on a scaled guiding brace (8), and the position of which is being fixable to the guiding brace (8) through a clasp (10).

4. Examination stand according to claim 1, comprising a standing plate (7) inserted into the standing ground for the stand-positioning of a person to be examined, being arrayed between the position plane (1) and the camera-/illumination plane (2) directly in front of the position plane (1).

5. Application of an examination stand according to one of the claims 1 - 4 within a computer-aided medical examination procedure for the recording, storage, and arithmetical processing of video-camera taken images of a body surface of a human individual, wherein within the procedural course a person to be examined is being positioned with the use of quantitative reproducible positioning means in a quantitatively reproducible position with respect to the course of time, wherein for the arithmetical image processing a quantitatively reproducible skin surface area is being selected, which is quantitatively taken through the positioning means, and the physical image parameters of which are being captured through a video camera, arithmetically stored and processed, and wherein through application of an image processing software the quantitative positioning reproducibility is being checked, and an arithmetical image analysis is being performed simultaneously on several to numerous distinguishable skin structures of the captured skin surface image in a way that the distinguishable skin structures are being assessed regarding their number, distribution, area dimension, and physical image structure as well as, with respect to the course of time, their changes of number, distribution, area dimension, and physical image structure, and are being optically labeled within the image, stored, arithmetically processed, and are being optically and arithmetically analysed in comparison with previously captured and stored images, wherein single structures, which have occurred newly over time, are being identified, and said structures as well as those single structures, that have changed and/or have remained unchanged over time in dimension, distribution, physical image structure as well as the positions of those single structures, which have been identified as missing over time, are being labeled within the image through optical enhancement.

6. Application according to claim 5, wherein an arithmetical image analysis is performed serially in consecution through a comparative optical and mathematical evaluation with respect to the course of time.

7. Application according to claim 5, wherein an optical labeling of the arithmetically processed distinguishable single skin surface structures is performed through numbering, colour enhancement, or graphical contouring, or through a combination of said labeling procedures.

8. Application according to claim 5, wherein optical and arithmetical analyses of the physical image parameters of the captured skin surface images are stored on data storage media, and are delivered graphically and arithmetically over a printer as a measurement protocol.

## Revendications

1. Niveau d'examen pour saisir, mémoriser et traiter directement en ordinateur des paramètres d'images physiques de la surface de la peau avec
- des moyens de positionnement (3, 4, 5) pour aboutir à une position du corps quantitativement reproductible pour des examens au cours du temps d'une personne à examiner,
- une caméra vidéo pour saisir les paramètres d'image physiques d'une partie de la surface de la peau,
- des moyens pour mémoriser et traiter les paramètres d'image physiques.
Pour aboutir à des positions du corps quantitativement reproductibles d'une personne à examiner, des éléments raccords sont joints à un support en cadre de telie manière qu'un plan de positionnement (1) et un plan de caméra/plan d'éclairage (2) sont disposés l'un après l'autre à une distance définie et joints par des éléments pour statifs (12) raccordés à angles droits avec le plan de positionnement (1) et le plan de caméra/plan d'éclairage (2). Les moyens pour positionner une personne à examiner dans une position quantitativement reproductible au niveau du plan de positionnement (1) contiennent, en direction verticale, à leur hauteur de disposition, des supports de régulateurs glissants et cadrés (3) ainsi qu'en direction horizontale des plaques terminantes réglables (5) dont les limitations de bord sont quantitativement saisies et traitées par un logiciel à traitement d'images dans une image prise par une caméra vidéo pour produire et contrôler la reproductibilité de positionnement. Dans la zone du plan de caméra/plan 'éclairage (2), il y a un support de caméra (9) réglable verticalement dans sa hauteur de disposition, fixé à une barre de guidage cadrée (8), disposée de manière verticale et centrée ainsi que des supports réglables verticalement à leur hauteur de disposition, fixés au support en cadre du plan de caméra/d'éclairage (2) pour tenir des éléments d'éclairage (11).

2. Niveau d'examen selon spécification 1: pour obtenir des positions de corps quantitativement reproductibles d'une personne à examiner dans la zone du plan de positionnement (1), il y a des supports de régulateurs glissants et cadrés (3), verticalement réglables à leur hauteur de disposition et munies de crochets de fermetures (4) à fixer au support en cadre. Ces régulateurs glissants sont munis de plaques terminantes latérales (5), dont la position horizontale est réglable sur le support de régulateur glissant (3) et qui peuvent être fixées au support par des leviers à serrage (6).

3. Niveau d'examen selon spécification 1: pour obtenir des conditions d'examen quantitativement reproductibles dans la zone du plan de caméra/plan d'éclairage (2), il y a un support de caméra (9) à déplacer verticalement à sa hauteur de disposition sur une barre de guidage cadrée (8), dont la position peut être fixée sur la barre de guidage (8) par un levier de fermeture (10).

4. Niveau d'examen selon spécification 1: pour positionner une personne à examiner, il y a une surface portante (7) insérée dans le plan de niveau qui est disposée entre le plan de positionnement (1) et le plan de caméra/plan d'éclairage (2), directement devant le plan de positionnement (1).

5. Utilisation des informations obtenues au niveau d'examen selon une des spécifications 1 à 4 dans le cadre d'un procédé d'examen médical, assisté par ordinateur, pour saisir, mémoriser et traiter en ordinateur des images prises par caméra vidéo d'une surface du corps d'une personne. La personne à examiner, tout en utilisant les moyens de positionnement quantitativement reproductibles, est positionnée dans une position quantitativement reproductible pour des examens au cours du temps. Pour le traitement d'images en ordinateur, une partie de la surface de la peau quantitativement reproductible est choisie moyennant les moyens de positionnement, dont les paramètres d'images physiques sont saisis par une caméra vidéo, mémorisés et traités en ordinateur. La reproductibilité de positionnement est contrôlée grâce à l'utilisation d'un logiciel pour traitement d'images. En même temps, une analyse d'images en ordinateur est réalisée de telle manière sur plusieurs caractéristiques structurelles à délimiter de la peau que les structures de la peau à délimiter peuvent être saisies par leur nombre, disposition, surface et structure d'image physique, marquées, mémorisées, traitées et analysées en ordinateur de manière optique, en les comparant à des images saisies et mémorisées antérieurement. Des structures individuelles récentes survenues au cours du temps peuvent être identifiées; ces structures ainsi que des structures ayant subi des modifications de dimension au cours du temps, n'ayant pas subi des modifications de dimension, ayant changé de disposition, ayant été modifiées dans leur structure d'image physique ainsi que les positions identifiées au cours du temps comme structures individuelles absentes peuvent être marquées par relèvement optique dans l'image.

6. Utilisation selon spécification 5: une analyse d'image en ordinateur est réalisée par une évaluation numérique, basée sur une comparaison optique, en séquence consécutive au cours du temps.

7. Utilisation selon spécification 5: un relèvement optique de structures individuelles délimitées, traitées en ordinateur, de la surface de la peau peut être réalisé par numérotage, couleur différente ou encadrement graphique ou par combinaison de ces procédés marqueurs.

8. Utilisation selon spécification 5: des analyses optiques et numériques de paramètres d'images physiques prises à partir d'images de la surface de la peau peuvent être mémorisées sur des supports de données et sorties sous forme graphique et numérique comme contrôle de mesures.
